Europäisches Patentamt

European Patent Office

Office européen des brevets

(11) Numéro de publication: **0 279 129 B1**

# FASCICULE DE BREVET EUROPEEN

(45) Date de publication de fascicule du brevet:
27.03.91

(51) Int. Cl.⁵: **A61F 2/08**

(21) Numéro de dépôt: 87402410.2

(22) Date de dépôt: **26.10.87**

(54) Dispositif d'ancrage sur un os d'une prothèse ligamentaire ou tendineuse munie de ganses d'extrémité, et ensemble de prothèse comportant un tel dispositif.

(30) Priorité: 28.10.86 FR 8614984

(43) Date de publication de la demande:
24.08.88 Bulletin 88/34

(45) Mention de la délivrance du brevet:
27.03.91 Bulletin 91/13

(84) Etats contractants désignés:
AT BE CH DE ES GB GR IT LI LU NL SE

(56) Documents cités:
EP-A- 0 145 492

MEDIZINISCH-ORTHOPAEDISHE TECHNIK,
vol. 101, no.3, mai-juin 1981, page 88, Stuttgart, DE; "InstruMed GmbH, medizinische Instrumente, Freiburg"

(73) Titulaire: **Cerol Bandeira, Maria de Lourdes**
**5 allée des Mouille Boeufs**
**F-92290 Chatenay Malabry(FR)**

Titulaire: **Porges, Antoine**
**19 Avenue Raymond Poincaré**
**F-75116 Paris(FR)**

(72) Inventeur: **Cerol Bandeira, Maria de Lourdes**
**5 allée des Mouille Boeufs**
**F-92290 Chatenay Malabry(FR)**
Inventeur: **Porges, Antoine**
**19 Avenue Raymond Poincaré**
**F-75116 Paris(FR)**

(74) Mandataire: **CABINET BONNET-THIRION**
**95 Boulevard Beaumarchais**
**F-75003 Paris(FR)**

## Description

L'invention se rapporte à un dispositif d'ancrage sur un os par une vis d'une prothèse ligamentaire ou tendineuse constituée de fibres et munie de ganses d'extrémité.

Des prothèses de ce genre sont décrites, notamment dans le document de brevet EP-A-0 051 954. Le document de brevet FR-A-2 569 989 enseigne d'utiliser des faisceaux de fibres polyarylamides dépourvues d'éléments protecteurs, afin d'induire une réaction à corps étranger au cours de laquelle se constituent des tissus fibreux néoformés capables de se substituer au moins partiellement à la prothèse.

Ces prothèses sont destinées, en remplacement de ligaments ou de tendons, à relier un os, soit à un autre os qui s'articule sur le premier (prothèse ligamentaire) soit à un muscle qui manoeuvre une articulation où coopère l'os (prothèse tendineuse).

L'ancrage de la prothèse sur l'os doit être bien entendu résistant dès la pose, même si l'ancrage se renforce naturellement par la suite, par "réhabitation" de l'extrémité de la prothèse par des tissus néoformés.

Par ailleurs il convient de conférer à la prothèse, lors de sa pose, une tension qui corresponde au mieux à la tension que l'organe, ligament ou tendon, que vient remplacer la prothèse, possède naturellement à l'état sain, pour une configuration déterminée de l'articulation desservie.

La technique usuelle, pour l'ancrage d'une telle prothèse sur un os, consiste (voir par exemple EP-A-0145492) à sertir sur une ganse d'extrémité un oeillet métallique, à passer une vis par le centre de cet oeillet dans cet oeillet, de faire pénétrer la vis dans un trou pratiqué à l'avance dans l'os à un emplacement approprié, pratiquement dans la zone d'insertion sur l'os de l'organe que vient remplacer la prothèse, et de serrer à fond cette vis pour forcer l'oeillet entre l'os et la tête de vis. Couramment la vis est du type vis autotaraudeuse, pour simplifier la préparation du trou dans l'os.

Un oeillet métallique avec des aspérités sur sa face en contact avec l'os pour assurer l'immobilisation est aussi connu (voir MEDIZINISCHORTHOPAEDISCHE TECHNIK, vol.101, Mai-juin 1981, page 88, stuttgart, DE; page 88, colonne 2)

On comprend que pour obtenir une tension optimale de la prothèse par ce processus, il faut déterminer avec une grande précision l'emplacement du trou, et qu'un écart ne peut pratiquement pas être corrigé. De plus, l'insertion de la vis dans son trou, alors que la prothèse est sous tension, présente certaines difficultés, la tension tendant à placer la vis en oblique par rapport à l'axe du trou.

L'invention a pour objet un dispositif d'ancrage de prothèse ligamentaire ou tendineuse qui permet d'ajuster la tension de la prothèse alors que celle-ci est déjà en place, et de bloquer le dispositif sur l'os en maintenant la tension réglée.

A cet effet, l'invention propose un dispositif d'ancrage sur un os, à l'aide d'une vis, d'une prothèse ligamentaire ou tendineuse constituée de fibres et munie de ganses d'extrémité, caractérisé en ce qu'il comporte une poulie avec une gorge apte à recevoir une ganse d'extrémité et percée, parallèlement à son axe, d'un passage de vis excentré, une face de la poulie étant munie d'aspérités propres à s'incruster dans l'os.

Pour poser la prothèse, on passe la poulie dans la ganse d'extrémité, celle-ci se logeant dans la gorge, le passage de vis étant au plus près du sommet de la ganse, on repère l'emplacement du trou de vis, on pratique ce trou, on passe la vis dans son passage dans la poulie dont la face munie d'aspérités est tournée vers l'os, on fait pénétrer la vis dans l'os jusqu'à amener la face de poulie munie d'aspérités au contact de l'os, on oriente la poulie autour de la vis pour tendre la prothèse au degré désiré, et on serre à fond la vis en sorte que les aspérités s'incrustent dans l'os. La poulie ne peut plus alors tourner et la tension de la prothèse est maintenue.

De préférence les aspérités sont des picots coniques disposés sur une circonférence centrée sur l'axe de poulie, et espacés les uns des autres de 120 %.

En disposition préférée la poulie est en deux parties à juxtaposition axiale, une partie présentant un bossage et l'autre un chambrage complémentaire du bossage pour permettre un emboîtement.

D'autres caractéristiques et avantages ressortiront d'ailleurs de la description qui va suivre, à titre d'exemple, en référence aux dessins annexés dans lesquels :

- la figure 1 est une vue de profil d'un dispositif selon l'invention;
- la figure 2 est une vue en plan, de dessous, de la pièce essentielle du dispositif selon l'invention;
- la figure 3 est une coupe suivant le plan III-III de la figure 2;
- la figure 4 est une vue en plan, de dessus, d'un dispositif selon l'invention engagé dans une prothèse pour ancrer celle-ci;
- la figure 5 est une vue analogue à la figure 3, d'une variante de l'invention.

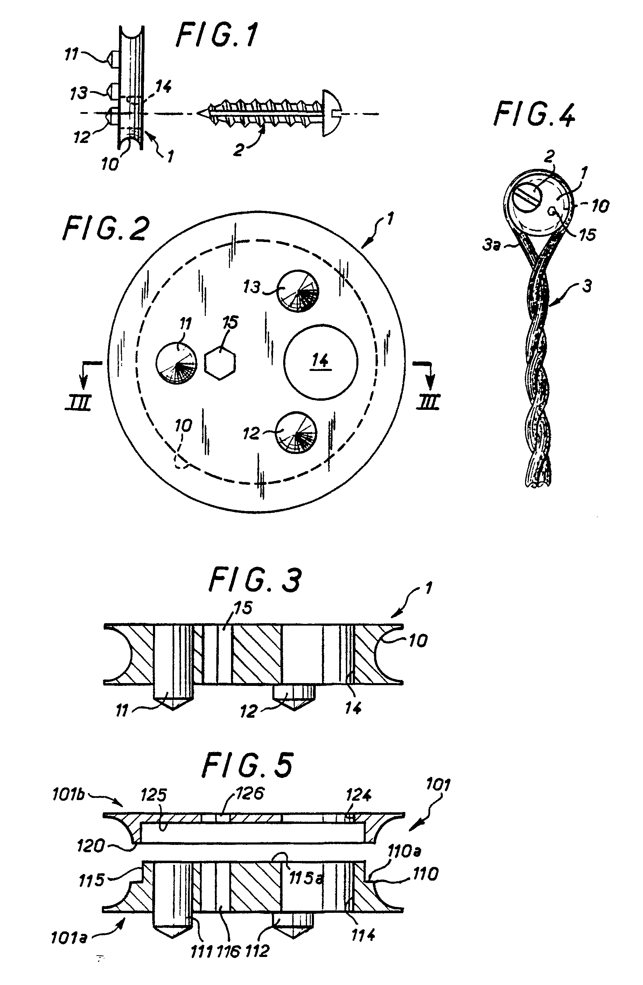

Selon le mode d'exécution choisi et représenté figures 1 à 3, le dispositif d'ancrage comporte une poulie 1 et une vis 2. La vis 2, est prévue pour fixer la poulie 1 sur un os, dans la zône d'insertion sur l'os de l'organe ligamentaire ou tendineux que la prothèse doit remplacer. C'est une vis classique en chirurgie prothétique, du type autotaraudant.

La poulie 1 est munie d'une gorge périphérique 10, à profil sensiblement semi-circulaire, qui doit recevoir la ganse d'extrémité d'une prothèse. La poulie 1 est traversée, parallèlement à son axe, par un passage 14 calibré pour laisser passer, avec du jeu, la vis 2. Ce passage 14 est excentré par rapport à la poulie 1.

Sur une face de la poulie 1, destinée à venir en contact avec l'os, sont disposés trois picots coniques 11, 12 et 13, usinés à l'extrémité de pions cylindriques emmanchés dans la poulie 1. Ces picots 11, 12 et 13 sont disposés sur une circonférence centrée sur l'axe de poulie, et espacés angulairement de 120°, en sorte de former les sommets d'un triangle équilatéral.

Dans l'exemple décrit ici, la poulie présente un diamètre extérieur de 16 mm, un diamètre en fond de gorge 10 de 13 mm et une épaisseur de 3 mm. Le rayon de gorge est de 1,3 mm, le diamètre du passage 14 de 4 mm pour accueillir une vis autotaraudeuse de 3,5 mm. Les pions cylindriques ont un diamètre de 2 mm et la saillie des picots 11, 12 et 13 au-delà de la face de la poulie est de 1,5 mm, le cône de picot ayant une hauteur de 0,5 mm.

Le rayon d'excentration, ou distance des centres de la poulie 1 et du passage 14, est d'environ 5 mm.

La poulie peut être exécutée en acier inoxydable, en titane ou en zirconium.

La prothèse utilisée de préférence en coopération avec le dispositif d'ancrage est constituée d'une torsade de fibre de polyarylamide résultant de la condensation de métaphénylènediamide et d'acide isophtalique, le polyarylamide étant connu sous le nom de marque de NOMEX ®, terminée à chaque extrémité par une ganse fourrée d'un fil de garniture en NOMEX également.

La torsade est constituée d'une nombre impair de brins formés par aller et retour d'un fil continu entre les deux ganses d'extrémité. Au cours de l'enroulement, effectué à tension constante, chaque brin reçoit individuellement une torsion d'un nombre déterminé de tours, toujours dans le même sens. Après l'arrêt de l'enroulement sur la ganse opposée à la ganse de départ, et fourrage des ganses par le fil de garniture, le détors des brins torsade ensemble deux torons constitués respectivement par les brins aller et les brins retour. Ce mode de réalisation assure une répartition égale des efforts sur tous les brins de la torsade, et un minimum d'effort de cisaillement des brins entre eux.

Si l'on se reporte à la figure 4 qui représente l'ancrage d'une prothèse 3, du type décrit ci-dessus, sur un os, on voit que la poulie 1 est engagée dans une ganse 3a d'extrémité de la prothèse 3, la ganse 3a se logeant dans la gorge 10 de la poulie 1, dont la forme correspond à celle de la ganse non déformée.

Après que la vis 2 ait été mise en place, mais non serrée, on a fait tourner la poulie 1, ici dans le sens inverse de celui des aiguilles d'une montre, pour conférer à la prothèse 3 une tension convenable. On a alors serré la vis 2 de sorte que les picots (ici invisibles) 11 à 13 s'incrustent dans la surface de l'os, en sorte d'empêcher un nouveau pivotement de la poulie 1, et de maintenir la tension de la prothèse 1 à la valeur déterminée.

Pour conférer à la prothèse une tension convenable, la poulie 1 comporte, parallèlement à son axe, un évidement 15 hexagonal pour l'insertion d'une clé six pans, type "Allen", la clé étant équipée d'un dispositif dynamométrique.

La poulie 101 représentée à la figure 5 est constituée en deux parties 101a et 101b, juxtaposables axialement de telle sorte que la poulie 101 présente la même configuration extérieure que la poulie 1 des figures 2 et 3.

La partie 101a comporte les picots 111 à 113, et un passage 114 pour la vis de fixation. On notera que le picot 113 est invisible sur la vue en coupe. La surface de juxtaposition débute en fond de gorge par une face annulaire plane 110a, qui entoure un bossage cylindrique 115 avec une tranche plane 115a.

La partie 101b comporte un passage 124 pour une vis de fixation, ce passage 124 venant en alignement avec le passage 114 dans la partie 101a. Cette partie 101b comporte une face de juxtaposition 120, plane, au centre de laquelle est usiné un chambrage 125, complémentaire du bossage 115. La profondeur du chambrage 125 est un peu supérieure à la hauteur du bossage 115, pour que les faces 110a et 120 soient juxtaposées avec précision et que, en fond de gorge 110, il n'existe pas de fente où pourrait se pincer un brin de la prothèse.

On remarquera que cette disposition facilite l'engagement de la ganse d'extrémité 3a (figures 4) dans la gorge de poulie 101, car il n'est plus nécessaire de faire passer la ganse d'extrémité par-dessus les bords de gorge.

Les deux parties 101a et 101b, comportent des passages hexagonaux 116 et 126, pour l'insertion d'une clé, et correspondant au passage 15 des figures 2 et 3.

Bien entendu, l'invention n'est pas limitée aux exemples décrits mais en embrasse toutes les variantes d'exécution, dans le cadre des revendications.

Il est notamment clair que les dimensions données dans la description n'ont pas de caractère limitatif.

**Revendications**

1. Dispositif d'ancrage sur un os, à l'aide d'une vis (2) d'une prothèse (3) ligamentaire ou tendineuse constituée de fibres et munie de ganses (3a) d'extrémité, caractérisé en ce qu'il comporte une poulie (1, 101) avec une gorge (10, 110) apte à recevoir un ganse d'extrémité (3a) et percée, parallèlement à son axe, d'un passage de vis (14; 114, 124) excentré, une face de la poulie (1, 101) étant munie d'aspérités (11, 12, 13; 111, 112, 113) propres à s'incruster dans l'os.

2. Dispositif selon la revendication 1, caractérisé en ce que la vis (2) est autotaraudeuse.

3. Dispositif selon l'une des revendications 1 et 2, caractérisé en ce que la gorge (10, 110) présente un profil sensiblement semi-circulaire

4. Dispositif selon une quelconque des revendications 1 à 3, caractérisé en ce que les aspérités sont des picots coniques (11, 12, 13; 111, 112, 113)

5. Dispositif selon la revendication 4, caractérisé en ce que les picots coniques (11, 12, 13; 111, 112, 113) sont disposés sur une circonférence centrée sur l'axe de poulie, à espacement angulaire de 120°.

6. Dispositif selon l'une des revendications 4 et 5, caractérisé en ce que les picots coniques (11, 12, 13; 111, 112, 113) forment l'extrémité de pions cylindriques rapportés.

7. Dispositif selon une quelconque des revendications 1 à 6, caractérisé en ce que la poulie (101) est en deux parties (101a, 101b) à juxtaposition axiale, avec une ligne de joint (110a, 120) suivant le fond de gorge (110).

8. Dispositif selon la revendication 7, caractérisé en ce qu'une première partie (101a) comporte un bossage (115) saillant cylindrique de même axe que la poulie et de diamètre inférieur à celui du fond de gorge, la seconde partie (101b) comportant un chambrage (125) adapté à s'emboîter sur le bossage (115) de la première partie (101a).

9. Dispositif selon les revendications 6 et 8 prises conjointement, caractérisé en ce que les pions portant les picots (111, 112, 113) sont rapportés dans la première partie (101a) de poulie.

10. Dispositif selon une quelconque des revendications 1 à 9, caractérisé en ce que la poulie (1, 101) comporte, parallèlement à son axe, un passage profilé (15, 116, 126) propre à recevoir une extrémité de clé.

11. Ensemble de prothèse comportant au moins un dispositif d'ancrage selon une quelconque des revendications 1 à 10, caractérisé en ce que la prothèse est constituée d'une pluralité impaire de brins entre deux ganses d'extrémité, les brins se succédant sur un fil unique d'un polyarylamide résultant de la condensation de phénylènediamide et d'acide isophtalique, et chaque brin ayant reçu une prétorsion déterminée égale.

12. Ensemble selon la revendication 11, caractérisé en ce que chaque ganse d'extrémité présente une garniture de fil.

**Claims**

1. A device for anchoring a ligament or tendon prosthesis (3) to a bone by means of a screw (2), said prosthesis being composed of fibres and being provided with end loops (3a), characterised in that it comprises a pulley (1, 101) with a groove (10, 11) able to receive an end loop [3a] and perforated, parallel to its axis, with an eccentric screw passage (14; 114, l24), one side of the pulley (1, 101) being provided with irregularities (11, 12, 13; 111, 112, 313) adapted to become incorporated into the bone.

2. A device according to claim 1, characterised in that the screw (2) is self-tapping.

3. A device according to either of claims 1 and 2, characterised in that the groove (10, 110) is of substantially semicircular section.

4. A device according to any of claims 1 to 3, characterised in that the irregularities are conical points (11, 12, 13, 111, 112, 113).

5. A device according to claim 4, characterised in that the conical points (11, 12, 13; 111, 112, 113) are disposed around a circumference centred on the pulley axis and are angularly spaced 120° apart.

6. A device according to either of claims 4 and 5, characterised in that the conical points (11, 12, 13; 111, 112, 113) form the end of separate cylindrical studs.

7. A device according to any one of claims 1 to 6, characterised in that the pulley (101) is in two axially juxtaposed parts (101a, 101b) with a joint line (110a, 120) following the bottom of the groove (110).

8. A device according to claim 7, characterised in that a first portion (101a) comprises a cylindrical projecting boss (115) having the same axis as the pulley and of a smaller diameter than the bottom of the groove, the second portion (101b) comprising a recess (125) adapted to fit on to the boss (115) of the first portion (101a).

9. A device according to claims 6 and 8 taken together, characterised in that the studs carrying the points (111, 112, 113) are inserted in the first pulley portion (101a).

10. A device according to any one of claims 1 to 6, characterised in that, parallel to its axis, the pulley (1, 101) comprises a profiled passage (15, 116, 126) adapted to receive a key end.

11. A prosthesis assembly comprising at least one anchoring device according to any one of claims 1 to 10, characterised in that the prosthesis comprises an odd number of strands between two end loops, the strands following a single thread of polyaryl amide formed by the condensation of phenylene diamide and isophthalic acid, each strand having been given an equal predetermined pretorsion.

12. An assembly according to claims 11, characterised in that each end loop has a thread lining.

## Ansprüche

1. Vorrichtung zur Befestigung einer aus Fäden bestehenden und am Ende mit Schlaufen (3a) versehenen Bänder- oder Sehnenprothese (3) mittels einer Schraube (2) an einem Knochen, **dadurch gekennzeichnet,** daß sie eine Scheibe (1, 101) mit einer Rille (10, 110) zur Aufnahme einer Endschlaufe (3a) aufweist, die parallel zu ihrer Achse eine exzentrische Bohrung (14; 114, 124) für den Durchtritt der Schraube und auf einer Seite der Scheibe (1, 101) Unebenheiten (11, 12, 13; 111, 112, 113), die in den Knochen einwachsen können, aufweist.

2. Vorrichtung nach Anspruch 1, dadurch gekennzeichnet, daß die Schraube (2) selbstschneidend ist.

3. Vorrichtung nach einem der Ansprüche 1 und 2, dadurch gekennzeichnet, daß die Rille (10, 110) ein im wesentlichen halbkreisförmiges Profil aufweist.

4. Vorrichtung nach einem der Ansprüche 1 bis 3, dadurch gekennzeichnet, daß die Unebenheiten konische Spitzen (11, 12, 13; 111, 112, 113) sind.

5. Vorrichtung nach Anspruch 4, dadurch gekennzeichnet, daß die konischen Spitzen (11, 12, 13; 111, 112, 113) auf einem zur Achse der Scheibe konzentrischen Umriss in einem Winkelabstand von 120° angeordnet sind.

6. Vorrichtung nach einem der Ansprüche 4 und 5, dadurch gekennzeichnet, daß die konischen Spitzen (11, 12, 13; 111, 112, 113) die Enden von angesetzten zylindrischen Stiften bilden.

7. Vorrichtung nach einem der Ansprüche 1 bis 6, dadurch gekennzeichnet, daß die Scheibe (101) aus zwei axial nebeneinander angeordneten Teilen (101a, 101b) mit einer dem Boden der Rille (110) folgenden Verbindungslinie (110a, 120) besteht.

8. Vorrichtung nach Anspruch 7, dadurch gekennzeichnet, daß ein erster Teil (101a) eine zylindrische vorspringende Wulst (115) mit gleicher Achse wie die Scheibe und geringerem Durchmesser als der des Bodens der Rille aufweist und der zweite Teil (101b) eine kammerartige Einsenkung (125) aufweist, welche sich über den Wulst (115) des ersten Teils (101a) stülpen kann.

9. Vorrichtung nach den zusammengenommenen Ansprüchen 6 und 8, dadurch gekennzeichnet, daß die die Spitzen (111, 112, 113) tragenden Stifte in den ersten Teil (101a) der Scheibe eingesetzt sind.

10. Vorrichtung nach einem der Ansprüche 1 bis 9, dadurch gekennzeichnet, daß die Scheibe (1, 101) parallel zu ihrer Achse ein Profilloch (15, 116, 126) zur Aufnahme eines Endes eines Schlüssels aufweist.

11. Prothese mit mindestens einer Befestigungsvorrichtung nach einem der Ansprüche 1 bis 10, dadurch gekennzeichnet, daß die Prothese aus einer ungeraden Mehrzahl von Fadenabschnitten zwischen zwei Endschlaufen besteht, wobei die Abschnitte an einem einzigen Faden aus einem Polyarylamid das durch Kondensation von Phenylendiamid und Isophthalsäure erhalten ist, aufeinander folgen und jeder Fadenabschnitt einen bestimmten gleichen Vordrall erhalten hat.

12. Prothese nach Anspruch 11, dadurch gekennzeichnet, daß jede Endschlaufe eine Fadengarnitur aufweist.

EP 0 279 129 B1

FIG.1

FIG.4

FIG.2

FIG.3

FIG.5

7